# EUROPEAN PATENT APPLICATION

(11) **EP 2 292 241 A1**
(43) Date of publication of application: **09.03.2011**
(21) Application number: 09749429.8
(22) Date of filing: 28.04.2009
(51) Int. Cl.: A61K 31/7048, C07D 407/12, A61K 9/00, A61K 9/08, A61K 9/12, A61P 29/00, A61P 9/00, A61P 35/00

(54) **A SCUTELLARIN DERIVATIVE, THE PREPARING PROCESS, THE PHARMACEUTICAL COMPOSITION AND THE USE THEREOF**

(30) Priority: 22.05.2008 CN 200810097680
(71) Applicant: Kunming Pharmaceutical Corp., Yunnan 650106 (CN)
(72) Inventor: ZHU, Huajie, Yunnan 650106 (CN); JIN, Yi, Yunnan 650106 (CN); XU, Shuguang, Yunnan 650106 (CN); YANG, Zhaoxiang, Yunnan 650106 (CN); PU, Junxue, Yunnan 650106 (CN)
(74) Representative: Detken, Andreas
(86) International application number: PCT/CN2009/071511
(87) International publication number: WO 2009/140886

(57) **Abstract**

A scutellarin derivative, 4',5,6-trimethoxy scutellarin, the preparing process, the pharmaceutical composition and the use thereof are provided. The compound is obtained by methyl-etherification of scutellarin as starting material, then separating by silica gel column. The compound of the present invention and the pharmaceutical composition thereof can be used for treating cardio-cerebro vascular disease, inflammation and tumor.

## Description

### FIELD OF THE INVENTION

The present invention relates to a new scutellarin derivative, its preparing process and a pharmaceutical composition containing the compound, and their application in cerebrovascular diseases, inflammation, and/or tumors.

### BACKGROUND OF THE INVENTION

*Erigeron Breviscapus* (also called "Herba erigerontis")is a plant of genus Erigeron, family Asteraceae. Breviscapine is a mixture (effective fraction) isolated from the whole plant of Erigeron Breviscapus, comprising mainly scutellarin (>90%) together with small amount of apigenin-7-O-glucuronside. Breviscapine has a function of activating blood circulation to dissipate blood stasis, expelling cold from the body surface, relaxing muscles and tendons and stimulating the circulation of the blood, which is often used clinically for the treatment of cardiovascular diseases. Currently commonly used breviscapine preparation in clinic is a preparation usually containing scutellarin as the major active ingredient which is extracted with ethanol and ethyl acetate. There are many reports of literatures on the treatment application of scutellarin in cardio-cerebro vascular diseases. Clinical application shows that scutellarin has the effect of increasing cardio-cerebro vascular blood flow, lowering vascular resistance, anti-aggregation of platelet and red blood cells, reducing blood viscosity and the like. The efficacy of scutellarin is reliable. In addition, there are a large amount of studies demonstrating that scutellarin has the effect of antiinflamation, antioxidation, and can also be used as a protein kinase inhibitor or be used for the treatment of diabeties, nephropathy, rheumatism, rheumatism and the like. Therefore, breviscapine is widely used clinically for treating cerebral thrombosis, cerebral infarction, paralysis after apoplexy of undetermined type, hypertension, cerebral embolism, multiple neuritis, paralysis caused by cerebral vascular (including chronic arachnoid) accident, coronary heart disease, angina, gouty arthritis and the like.

Since scutellarin is sparingly soluble in water, and has a short biological half life and a low absolute biological availability, these limit its application in medicine field. In order to improve the above shortcomings of scutellarin and expand its application, structure modifications thereon have been performed to find new type of active compounds. It was reported in the literature (Eur. J. Pharm. Sci, 2006,29,5,385-393) that prodrug synthesis of ethyl esterification, benzyl esterification and hydroxyacetylamide esterification has been made on the glucoside carboxyl group of the scutellarin, and found that the solubility of hydroxyacetylamide ester of scutellarin in buffer solution (pH=4.2) and in water was increased by nearly 10 and 35 times respectively, compared with those of scutellarin.

However, existing scutellarin or its derivatives has a poor solubility and low biological availability, thus a desirable therapy effect can not be achieved.

### SUMMARY OF THE INVENTION

The present inventor found that a scutellarin derivative having a new structure can be obtained by introducing methyl groups on phenol hydroxyl groups of flavone structure of scutellarin.

Therefore, one purpose of the present invention is to provide a new scutellarin derivative having a structure of formula (I) as the following:

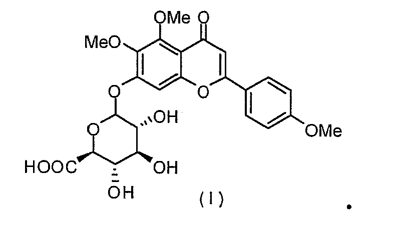

The scutellarin derivative showed in formula (I) can also be in the form of a salt thereof, especially a pharmaceutically acceptable salt thereof, including a salt with an inorganic or organic base. Said inorganic base is for example an alkali compound of alkali metals or alkali earth metals, especially sodium hydroxide (NaOH), potassium hydroxide (KOH), calcium hydroxide (Ca(OH)₂), and the like. Said organic base is for example amine, especially L-lysine, L-arginine, triethylamine and the like. Said salts can be obtained by any conventional methods known in the field.

Another purpose of the present invention is to provide a preparing process of the scutellarin derivative of formula (I) according to the present invention, comprising the following steps:
a) dissolving breviscapine in an organic solvent;
b) adding a methyl-etherification reagent and a base to the above organic solvent under stirring at 0 to 40 °C , preferably at room temperature;
c) extracting and isolating the product obtained from step b) to obtain the scutellarin derivative of formula (I).

In the context of the invention, room temperature means any temperature in the range of 15 to 25°C.

For ease of description, the compound of formula (I) (the chemical name is 4',5,6-trimethoxylscutellarin) can also be referred to as A1 hereinafter.

Another purpose of the present invention is to provide a pharmaceutical composition for preventing, improving and treating cardio-cerebro vascular diseases, inflammation and/or tumors, characterized in that the pharmaceutical composition comprises therapeutically effective amount of scutellarin derivative of formula (I) according to the present or a pharmaceutically acceptatble salt thereof as an active ingredient, together with a pharmaceutically acceptatble carrier.

A further purpose of the present invention is to provide use of the scutellarin derivative of formula (I) or a pharmaceutically acceptatble salt thereof according to the present invention in the manufacture of a medicament for preventing, improving or treating cardio-cerebro vascular diseases, inflammation and/or tumors.

Compound A1 of the present invention has a good solubility and a high biological availability, and can achieve a reliable efficacy. Therefore, compound A1 of the present invention or a pharmaceutical composition containing compound A1 can be effectively used for preventing, improving and treating cardio-cerebro vascular diseases, inflammation and/or tumors, instead of existing scutellarin or its derivatives.

Furthermore, compound A1 according to the present invention can be obtained by using raw material breviscapine extracted in industrial scale as the starting material, through methyl-etherification reaction and followed by silica column isolation. Therefore, the scutellarin derivative (A1) of the present invention is ready to be prepare, and possesses the advantages of mild reaction conditions, low cost, easy to operate and suitable for industrialization and the like, and thus can achieve an especially outstanding economic benefit.

### DETAILED DESCRIPTION OF THE INVENTION

In some embodiments of the present invention, a new scutellarin derivative is provided, which structure is showed as formula (I):

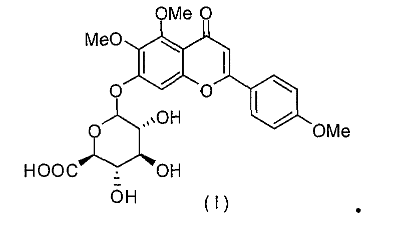

The scutellarin derivative showed in formula (I) can also be in the form of a salt thereof, especially a pharmaceutically acceptable salt thereof, including a salt with an inorganic or organic base. Said inorganic base is for example an alkali compound of alkali metals or alkali earth metals, especially sodium hydroxide (NaOH), potassium hydroxide (KOH), calcium hydroxide (Ca(OH)₂), and the like. Said organic base is for example amine, especially L-lysine, L-arginine, triethylamine and the like. Said salts can be obtained by any conventional methods known in the field.

In other embodiments of the present invention, a process for preparing the scutellarin derivative (A1) of formula (I) according to the present invention is provided, comprising the following steps:
a) dissolving breviscapine in an organic solvent;
b) adding a methyl-etherification reagent and a base to the above organic solvent under stirring at 0 to 40 °C, preferably at room temperature;
c) extracting and isolating the product obtained from step b) to obtain A1.

In some preferred embodiments of the present invention, said organic solvent in step a) may be single solvent, or a mixed solvent of two or more solvents. Said organic solvent may be selected from the group of N,N-dimethylformamide, dimethylsulfoxide, dichloromethane, dichloroethane and any combination thereof. The amount of the solvent is not limited specifically, provided that breviscapine can be fully dissolved. Preferablly, according to the amount in milliliter of the solvent used, the amount of the solvent used is 10-100 ml per gram of breviscapine, more preferably 20-80 ml per gram of breviscapine, most preferably 30-50 ml per gram of breviscapine.

In some specific embodiments, step a) is performed by dissolving breviscapine in a mixed solvent of N,N-dimethylformamide (DMF) and dichloromethane, more preferably a mixed solvent of N,N-dimethylformamide (DMF) and dichloromethane in a volume ratio of 10:1 to 10:5, preferably 10:2 to 10:4, more preferably 10:3. Alternatively, dimethylsulfoxide (DMSO) may be used to replace N,N-dimethylformamide (DMF), and/or dichloroethane may be used to replace dichloromethane, and other similar replacements are also possible.

In some preferred embodiments of the present invention, there is no extra limitation on the methyl-etherification reagent in step b), for example, it can be selected from halomethane, dimethyl sulfate and dimethyl carbonate, preferably halomethane, such as methyl iodide, methyl bromide and the like, most preferably methyl iodide, and the amount thereof in molar ratio is 1-10:1, preferably 3-5:1, more preferably 4:1, based on the amount of the reactant.

In some preferred embodiments of the present invention, there is no extra limitation on the base in step b), it is preferably M₂CO₃ or MHCO₃, wherein M is Na, K or Li, and the amount in molar ratio of the base used is 1-15:1, preferably 3-10:1, more preferably 5-8:1, based on the amount of the reactant.

In some preferred embodiments of the present invention, the exaction and isolation in step c) can be performed by chromatography, for example, isolation via chromatography on a silica or alumina column using chloroform, dichloromethane, ethyl acetate, methanol, petroleum ether or a mixture thereof as eluent. In some specific embodiments, step c) is performed by obtaining A1 by exacting and separating the product obtained in step b) through silica column separation using ethyl acetate/methanol as an eluent in a volume ratio of of 1-15:1, preferably 3-10:1, more preferably 6-8:1.

Compound A1 of the present invention or a pharmaceutically acceptable salt thereof may be formulated into a pharmaceutical composition and be administrated to a mammal for example human in various forms suitable for the route of administration selected, for example by oral, parenteral, intravenous, intramuscular, topical or subcutaneous route.

Therefore, in other embodiments of the present invention, a pharmaceutical composition for preventing, improving or treating cardio-cerebro vascular diseases, inflammation and/or tumors is provided, characterized that the pharmaceutical composition comprises therapeutically effective amount of scutellarin derivative of formula (I) according to claim 1 or a pharmaceutically acceptatble salt thereof as an active ingredient, together with a pharmaceutically acceptatble carrier.

In the present invention, said pharmaceutically acceptatble carrier means conventional pharmaceutical carrier in pharmaceutical field, such as excipients, fillers, binders, disintegrating agents, lubricants, antioxidants, coating agents, coloring agents, fragrances, surfactants, and the like.

The pharmaceutical composition of the present invention may be formulated into conventional preparations including oral preparations, injections or spray (such as nasal spray) according to conventional preparation methods. Corresponding carriers can be selected according to different dosage forms of the pharmaceutical composition. The preparation of corresponding dosage forms may be achieved according to conventional prior art in the field of formulation, and coating may be performed, if desired.

For example, compound A1 of the present invention or a pharmaceutically acceptable salt thereof may be combined with pharmaceutically acceptatble carriers for system administration, for example oral administration. They can be encapsuled into hard shell or soft shell gelatin capsules, or can be pressed into tablets, or can be directly added to the diet of patients. For oral administration, the active compounds can be combined with one or more carriers and administered in the forms such as tablets, buccal tablet, lozenge, capsule, elixir, suspension, syrup, wafer and the like.

The preparations including tablets, lozenges, pills and capsules can also contain the following components: binders, such as tragacanth gum, arabic gum, corn starch or gelatin; excipients, such as dicalcium phosphate; disintegrating agents, such as corn starch, potato starch, alginate and the like; lubricants, such as magnesium stearate; and sweeteners, such as sucrose, fructose, lactose or aspartame; or flavoring agent, such as mint, wintergreen oil; or other perfumes can be added. When the unit dosage form is a capsule, in addition to the above-mentioned types of materials, it can also contain liquid carriers such as vegetable oil or polyethylene glycol. A variety of other substances can exist as a coating or to change the physical form of the solid unit dosage forms. For example, the preparations including tablets, pills or capsules can be coated with gelatin, wax, shellac or sugar. Syrup or elixir can contain active compounds, sucrose or fructose as a sweetener, methylparaben and propylparaben as a preservative, dyes and flavoring agent such as cherry or orange flavor. It should be noted that, any of the substances used in the preparation of any unit dosage form should be pharmaceutical acceptable and essentially non-toxic in terms of the amount used. In addition, the active compounds can be added to the sustained-release preparations and devices.

The active compounds can also be administered via infusion, intravenous injection or intraperitoneal injection. The solution of the active compounds or salts thereof can be prepared in water optionally mixed with non-toxic surfactants. Furthermore, the dispersion can also be prepared in glycerol, liquid polyethylene glycol, triacetin and mixtures thereof and in oil. In ordinary conditions of storage and use, these preparations may contain preservatives to prevent microbial growth.

Dosage forms suitable for injection or infusion may include sterile aqueous solution or dispersions or sterile powder containing the active ingredient, which are suitable for temporary preparation of sterile injectable or infusion solution or dispersions, optionally encapsulated in the liposomes. In all cases, the final dosage form should be sterile, flowable and stable in the conditions of preparation and storage. Liquid carrier or vehicle can be a solvent or liquid dispersion medium containing for example water, ethanol, polyols (such as glycerol, propylene glycol, liquid polyethylene glycol, etc.), vegetable oil, non-toxic glyceride and an appropriate mixture thereof. For example, adequate flowability can be maintained by formation of liposomes, maintaining the required particle size in the case of dispersion or using surfactants. Various antibacterial agents and antifungal agents (for example, parabens, chloro-butanol, phenols, sorbic acid, thimerosal, etc.) to prevent microbial growth. In many cases, an isotonic agent such as sugar, buffer or sodium chloride is preferably comprised. An absorption-delaying agent such as aluminum monostearate or gelatin may be added to injectable compositions in order to prolong the absorption of the composition.

A sterile injectable solution can be prepared through mixing the required amount of active compounds with various other ingredients listed above in an appropriate solvent, followed by filtration sterilization, as required. In the case of preparing sterile powders for sterile injectable solution, vacuum drying and freeze-drying techniques are the preferred methods. A powder containing the active ingredients and any additional desired component present in the above sterile filtered solution is obtained.

For topical administration, the compound of the present invention may be administrated in pure form (i.e. in case of liquid). However, the compound of the present invention may be administrated to the skin as a composition or preparation in combination with dermalogically acceptable carriers which can be solid or liquid.

Solid carriers that can be used include finely divided solid, for example, talc, microcrystalline cellulose, silica, alumina and the like. Liquid carriers that can be used include water, dimethylsulfoxide (DMSO), alcohols or diols or water-alcohol/diols mixture, in which the compound of the present invention can be dissolved or optionally dispersed in an effective level with the aid of a non-toxic surfactant. Adjuvants for example fragrance and additional antimicrobe agent so as to optimize the properties with respect to given uses. Liquid composition obtained can be administrated by absorption pad or by immersion band or other dressings, or be sprayed at affected area using a pump nebulizer. Preferablly, it is administrated in the form of nasal spray.

It is also possible to use thickening agent with liquid carriers, said thickening agent is for example synthetic polymers, aliphatic acids, salt and ester of an aliphatic acid, aliphatic alcohols, modified cellulose or modified minerals, to form a spreadable paste, gel, ointment or soap and the like, which can be directly applied onto the user's skin.

It is preferable that the pharmaceutical composition or preparation of the present invention contain the active compound in a weight ratio of at least 0.1%, preferably 0.5%-99.5%, more preferably 1.0%-90.0%, based on the total weight of the pharmaceutical composition or preparation. The percent of the pharmaceutical composition or preparation may vary and may advantageously account about 2% to about 60% of the weight of the given unit dosage form.

The pharmaceutical composition of the present invention may be used according to the conventional methods of various types of formulations, and the dosage may be changed according to the route of administration, the age and body weight of patients, the type and severity of the diseases to be treated. For example, for oral administration, single dosage is 10mg-100mg active compound and daily dosage is 20mg-300mg active compound; for injection route, single dosage is 5mg-80mg active compound, once or twice daily. Administration should be made based on the individual conditions of patients, typically daily therapeutic dosage is 50mg-200mg active compound for oral administration, 20mg-100mg active compound for injection administration, and 15mg-60mg active compound for preventive oral administration.

In a further embodiment of the present invention, use of scutellarin derivative of formula (I) according to the present invention or a pharmaceutically acceptatble salt thereof in the manufacture of a medicament for preventing, improving or treating cardio-cerebro vascular diseases, inflammation and/or tumors is provided.

Said cardio-cerebro vascular diseases is for example cerebral thrombosis, cerebral infarction, paralysis after apoplexy of undetermined type, paralysis caused by cerebrovascular accident including chronic arachnoid membrane, angina and the like.

Said inflammation is for example polyneuritis, gouty arthritis and the like.

Said tumors include benign tumors and malignant tumors, for example, adenoma, lipoma, osteoma, skin cancer, stomach cancer, intestinal cancer, lung cancer, and the like.

The present inventor assessed the effect of A1 on acute cardio- and cerebral-ischemia by determining multiple indicators of pharmacodynamics using an animal model similar to human diseases. The results of animal experiments showed that, A1 has a prominent protection effect on myocardial ischemia under experiment conditions, and high dosage group of 40mg/kg of A1 showed a better effect over 40mg/kg of breviscapine tablets and 50mg/kg of nimodipine tablet. No toxic or adverse effect was found when the compound of the present invention was used in a high dosage, suggesting it is safety.

The preparing process, pharmacological actions of A1 and the preparing process of the conventional preparations of the pharmaceutical composition according to the present invention will be described in detail hereinafter. Those skilled in the art will fully understand the present invention with the help of the following description. However, it should be noted that the present invention will not be limited thereto.

### Preparative Example 1 the preparation of 4',5,6-trimethoxy_ scutellarin(A1)

Dissolving 5 g (10.8mmol) of scutellarin (manufactured by Kunming Pharmaceutical Group Co. Ltd.) in 100ml of mixed solvent of DMF and dichloromethane in a volume ratio of 10:2, then 4.6g of methyl iodide and 7.5g (54mmol) of potassium carbonate was added and stirred at room temperature for 2 days. TLC (thin layer chromatography) showed that the reaction was complete. 50 ml of 1N Hydrochloric acid was added and extraction with 50 ml ethyl acetate was performed 3 times. The organic phase was pooled and dried on anhydrous sodium sulphate. The organic solvent was evaporated to dryness under rotation. Extraction and isolation was performed on silica column (ethyl acetate : methanol= 10:1) and 3.1g of light yellow solid was obtained (yield: 60%). H¹NMR (400MHz, CDCl₃) δ7.78 (d, 2H), 6.92 (d, 2H), 6.60 (s, 1H), 6.50 (s, 1H), 4.94 (d, 1H), 4.01 (d, 1H), 3.83 (s, 3H), 3.81 (s, 3H), 3.71 (s, 3H), 3.47-3.67 (m, 5H); C¹³NMR (100MHz, CDCl₃) δ181.76, 181.68, 168.14, 163.59, 163.54, 161.79, 155.15, 152.38, 152.10, 151.62, 132.25, 127.28, 122,17, 113.62, 105.97, 102.82, 100.13, 93.63, 75.02, 74.77, 71.90, 70.31, 60.00, 54.63, 51.54; MS(ESI) M⁺ 504.

### Experiment 1 Pharmacological study on the protection effect of A1 on cardio- and cerebral-ischemia

A mass of pharmacological studies proved that scutellarin has the effects of expending cerebral blood vessels, lowering cerebral vascular resistance, increasing cerebral blood flow, improving microcirculation, increasing blood-brain barrier permeability, enhancing the body immune macrophages, and against cerebral ischemia and hypoxia caused by pituitrin, and against the adenosine diphosphate(ADP)-induced platelet aggregation. Breviscapine is widely used clinically for treating cerebral thrombosis, cerebral infarction, paralysis after apoplexy of undetermined type, hypertension, cerebral embolism, multiple neuritis, paralysis caused by cerebral vascular (including chronic arachnoid) accident, coronary heart disease, angina, gouty arthritis and the like.

The activities of compound A1 of the present invention will be illustrated by the following animal pharmacological experiments, and the prominent therapeutic effects thereof will be proved by comparative study with breviscapine and nimodipine.

An animal model which is similar to human diseases and has a good repeatability is a basis for evaluation of drug therapeutic effect. Two animal models similar to human diseases were adopted in the following biological assays to determine multiple pharmacodynamics indicators, and evaluate the effect of A1 on cardio- and cerebral-ischemia.

### 1. Materials

### 1.1 drugs and reagents

Pure A1 synthesized above; breviscapine commercial available, 20mg/tablet, batch number 20070418, manufactured by Pharmaceutical Factory of Yunnan Institute of Materia Medica; nimodipine (Nidaer Pian), 20mg/tablet, manufactured by Tianjin Central Pharmaceutical Co. Ltd., batch number 070301. All of the above samples were formulated into suspensions of desired concentration with 0.5% sodium carboxymethyl cellulose (CMC-Na) for the administration of rat via gavage.

red tetrazoline (TTC), imported, AMRESCO subpakage, batch number 0765, manufactured by Shanghai Sangon Biotechnology Company; adenosine 5'-diphosphate sodium(ADP), batch number A2754, manufactured by Sigma Company, imported and loaded separately by Beijing Dingguo bio-tech Co. Ltd.

AST (serum aspartate aminotransferase)(ultraviolet dynamics method, batch number 0704151), LDH((serum)lactate dehydrogenase) (ultraviolet dynamics method, batch number 0704102. CK-MB (creatine kinase isoenzyme of myocardium type) (selective inhibition kinetics method, batch number 100412), TT (thrombin time) (batch number 0504052), PT (prothrombin time) (batch number 0604161). APTT (activated partial thromboplastin time) (batch number 0604131) and FIB (fibrinogen) (batch number 060405) kit, all of above were manufactured by Sichuan Maker Science And Technology Co., Ltd.

### Animals

Clean animal SD rat, mail, body weight 240-385g, license number: SCXK(Hu)2003-0002, from Shanghai Sippr-bk laboratory animal Co. Ltd., purchased on commission by Kunming Medical College Experimantal Animals Center.

### Instruments

TM1024 automatic biochemical analyzer, Tokyo, Japan Co., Ltd.; Centrifuge 5810R refrigerated centrifuge, Eppendoff AG, Germany; BS 110 electronic analytical balance, Sartorius AG; Millipore ultrapure water system, Millipore Corporation,USA; 6951D Electrocardiograph, Shanghai Nihon Kohden Medical Electronic Instrument Co., Ltd.; C2000-4 coagulometer and LBY-NS platelet aggregation device, Beijing Precil Group Co. Ltd..

### Statistical Analysis

The results were expressed as X̅±SD, normal distribution of data was tested with t-test, skewed distribution of data was tested with rank sum test.

### 2 Method

### 2.1 Experiment of myocardial ischemia caused by coronary artery ligation in rats

### 2.1.1 grouping and modeling

Male SD rats of 330~385g were divided at random into sham group, model group, breviscapine tablet of 40mg/kg group, A1 20mg/kg group and A1 40mg/kg group. Animals of each group were gavage once daily for consecutive 5 days in predetermined dosage, for blank group and model group, the rats were given same volume of 0.5% CNC-Na in 10ml/kg body weight. At 30 minute after the last administration, the animals were anesthetized with 12% chloral hydrate in 350 mg/kg via intraperitoneal injection, then a thoracotomy was performed to render the heart exposed. The left anterior descending coronary artery was found between pulmonary artery cone and left ventricular and was ligated at 2-3mm to the root. Reposited the heart and closed the thoracic cavity. For the sham group, the suture was merely placed but not ligated.

### 2.1.2 detection indicators

II lead limb ECGs before ligation and at 5min, 30 min, 1h, 2h, 4h and 24h after ligation were recorded, respectively. The change of S-T segment (J point) was measured. After 24h ECG was recorded, blood was collected in duplicate via carotid artery intubation. For one sample, serum was separated and the activity of AST, LDH and CK-MB was determined according to the method of the kit; for another sample, it was treated with 3.8% sodium citrate in 1:9 for the purpose of anticoagulation, centrifuged at 1000rpm for 5 minutes to prepare platelet rich plasma (PRP), and centrifuged at 3500rpm for 10 minutes to prepare platelet poor plasma (PPP). The rate of platelet aggregation induced by ADP-2Na was determined according to turbidimetry, the final concentration of ADP was 6mol/L; TT, PT, APTT and FIB was determined according to the method of the kit, respectively. The heart was removed quickly after blood taking, and placed into ice cold saline solution to pump out deposit blood in thoracic cavity. The atria and adipose tissue were removed, and water was adsorbed to dryness. The ventricle was weighed, cut into 5 pieces along the coronal, then incubated and stained for 10 minutes in 1% TTC solution at 37°C in darkness. The normal myocardial tissue showed red after stain, while infarcted tissue showed white. The infracted part was cut and weighed. The percentage of infracted part accounting for the total weight of ventricle was calculated, which was the range of myocardial infarction.

### 2.2 focal cerebral ischemia experiment induced by MCAO (middle cerebral artery occlusion) in rats

### 2.2.1 grouping and modeling

Male SD rats, weighing 240~290g, grouping and dosages were as above. Additionally, a positive control group, i.e., nimodipine 50mg/kg group was set up. Animals of each group were gavage once daily for consecutive 5 days in predetermined dosage. At 30 minute after the last administration, the animals were anesthetized with 12% chloral hydrate in 350 mg/kg via intraperitoneal injection. The animals were kept left lateral position and the skin was cut along the middle point between right ear and eye line. Temporalis was separated and cheekbone was sheared. A holl was drilled with a dental drill in front of zygomatic root and MCA was exposed. The MCA was hooked with a needle between the vein and olfactory tract in the brain, then was broke, after bleeding was stopped with a cotton ball, muscle and skin layer was sutured in layer. For the sham group, except for no breaking of MCA, other steps were same.

### 2.2.2 Indicators

The animals were scored on neurological functions at 6 hour and 24 hour after modeling, according to behavior such as movement. Such score was used as an indicator of brain dysfunction. The scoring was performed using blindness, i.e., the scorer did not know the administration's case. The standards of grading were as follows;

| symptom | score on behavior disorder |
|---|---|
| Lift the tail of rat off the ground about 1 foot, carpal flexion, elbow flexion or shoulder internal rotation appeared on surgery contralateral forelimb, or carpal flexion, elbow flexion and shoulder internal rotation appeared | 1-4 |
| Place the animal on a flat ground and check resistance by pushing both shoulders inside | 1-3 |
| Place the animal on a metal net plane and observe the tension of both forelimbs | 1-3 |
| Place the animal on a flat ground and observe whether there is turning around | 1-2 |
| Full score | 12 |

The full score of the above standards is 12. The higher the score is, the more severe the behavior disorder is.

24 hour after modeling and after neurological scoring, blood was taken via carotid artery intubation in duplicate. For one sample, serum was separated and the activity of AST and LDH was determined according to the method of the kit; for another sample, it was treated with 3.8% sodium citrate in 1:9 for the purpose of anticoagulation, centrifuged at 1000rpm for 5 minutes to prepare platelet rich plasma (PRP), and centrifuged at 3500rpm for 10 minutes to prepare platelet poor plasma (PPP). The rate of platelet aggregation induced by ADP-2Na was determined according to turbidimetry, the final concentration of ADP was 6mol/L; TT, PT, APTT and FIB was determined according to the method of the kit, respectively. The brain was removed quickly by decollation after blood taking, and then placed into ice cold saline solution to remove olfactory bulb, cerebellum and brainstem. The brain was weighed after water being absorbed to dryness, and then cut into 5 pieces along the coronal, placed into a 5ml solution containing 4% TTC and 0.1ml K₂HPO₄ (1mol/L) and incubated for 30 minutes at 37°C and in darkness. During the course every 7 to 8 minutes a turning was made. The normal brain tissue showed rose red after stain, while infarcted tissue showed white. The infracted part was cut and weighed. The percentage of infracted tissue accounting for the total weight of bilateral cerebral hemispheres was calculated by weight area method, which was the range of cerebral infarction.

### Results

### 3.1 Effect on myocardial ischemia induced by coronary artery ligation in rat

### 3.1.1 Effect on S-T segment of ECG of myocardial ischemia

For modeling group, after coronary artery ligation, there was a prominent increase or decrease on the S-T segment (J point) of ECG, while there was no substantial change for the shame group. These showed that the modeling in the experiment was successful. For A1 high dosage group, the changes of S-T segment of ECG at different time points were significantly decreased, showing that high dosage of A1 had a prominent improvement on the ECG of myocardial ischemia; for A1 low dosage group, low dosage of A1 merely showed an improvement on the ECG of myocardial ischemia at 5 minutes, 30 minutes and 24 hours. The results were summarized in table 1.

**Table 1 Effect of A1 on S-T segment of ECG of myocardial ischemia in rats**

| group | dosage (/kg) | number of animals | change of S-T segment after ligation (X±SD, mv) | | | | | |
|---|---|---|---|---|---|---|---|---|
| | | | 5min | 30min | 1h | 2h | 4h | 24h |
| sham | 10ml | 10 | 0.06±0.06 | 0.05±0.05 | 0.04±0.05 | 0.06±0.04 | 0.06±0.05 | 0.01±0.02 |
| modeling | 10ml | 11 | 0.37±0.12^{ΔΔ} | 0.24±0.09^{ΔΔ} | 0.14±0.08^{ΔΔ} | 0.16±0.08^{ΔΔ} | 0.16±0.10^{ΔΔ} | 0.18±0.14^{ΔΔ} |
| breviscapine tablet | 40mg | 12 | 0.15±0.14^{**} | 0.09±0.08^{**} | 0.07±0.06^{*} | 0.07±0.07^{**} | 0.08±0.06^{*} | 0.02±0.03^{**} |
| A1 | 40mg | 12 | 0.14±0.12^{**} | 0.06±0.06^{**} | 0.05±0.04^{**} | 0.05±0.04^{**} | 0.06±0.05^{*} | 0.02±0.03^{**} |
| A1 | 20mg | 11 | 0.17±0.11^{**} | 0.12±0.09^{**} | 0.11±0.08 | 0.11±0.07 | 0.11±0.08 | 0.07±0.08^{*} |

| | | | | | | | | |
|---|---|---|---|---|---|---|---|---|
| Compared with the sham group: ^{ΔΔ}P < 0.01; Compared with the modeling group: *P<0.05, **P<0.01. | | | | | | | | |

### 3.1.2 Effect on the enzymology of myocardium with ischemia in rats

For modeling group, the activity of serum AST, LDH and CK-MB in rats were increased in different degrees, especially for CK-MB and LDH, the increase of activity was more prominent. These showed that the modeling of myocardial damage was successful. Both dosages of A1 significantly decreased the release of CK-MB and LDH and had a trend of lowing AST, showed a prominent effect of lowing myocardial cells damage; for both of the administration groups, the range of myocardial infarction were significantly smaller than that of modeling control group, and decreased by 60.87% and 45.65%, respectively, compared with the modeling group. The results were summarized in table 2.

**Table 2 Effect of A1 on the range of myocardial infarction and enzymology of rats**

| group | dosage (/kg) | number of animals | myocardial enzymology (X±SD, ku/L) | | | range of myocardial infarction | |
|---|---|---|---|---|---|---|---|
| | | | AST | LDH | CK-MB | (X±SD, %) | rate of decrease |
| sham | 10ml | 10 | 0.52±0.21 | 0.82±0.21 | 0.65±0.26 | 0.19±0.27 | - |
| modeling | 10ml | 11 | 0.72±0.35 | 1.08±0.27^{Δ} | 2.06±1.30^{ΔΔ} | 6.44±3.62^{ΔΔ} | - |
| breviscapine tablet | 40mg | 12 | 0.68±0.33 | 0.94±0.42 | 0.92±0.64^{*} | 3.09±1.58^{*} | 52.02 |
| A1 | 40mg | 12 | 0.61±0.20 | 0.61±0.24^{**} | 0.80±0.68^{*} | 2.52±2.16^{*} | 60.87 |
| A1 | 20mg | 11 | 0.61±0.26 | 066±0.32^{**} | 0.71±0.45^{**} | 3.50±2.68^{*} | 45.65 |

| | | | | | | | |
|---|---|---|---|---|---|---|---|
| Compared with the sham group: ^{Δ}P < 0.05, ^{ΔΔ}P < 0.01; Compared with the modeling group: *P<0.05, **P < 0.01. | | | | | | | |

### 3.1.3 Effect on platelet aggregation and coagulation of the rats with myocardial ischemia caused by coronary artery ligation

After modeling of myocardial ischemia caused by coronary artery ligation in rats, platelet aggregation rate was significantly increased, ATPP time had a trend of decrease, and the content of fibrinogen was increased significantly. Each administration group was able to significantly inhibit platelet aggregation induced by ADP, and had a trend of increasing TT and APTT time. A1 group also had a trend of decreasing the content of fibrinogen. The results were summarized in table 3.

**Table 3 Effect of A1 on coagulation and platelet aggregation caused by coronary artery ligation in rat**

| group | dosage (/kg) | number of animals | indicators of coagulation | | | | rate of platelet aggregation (%) |
|---|---|---|---|---|---|---|---|
| | | | TT (s) | PT (s) | APTT (s) | FIB (g/L) | |
| sham | 10ml | 10 | 77.14±25.12 | 23.74±6.65 | 85.95±30.16 | 3.57±0.49 | 49.00±6.34 |
| modeling | 10ml | 11 | 72.37±40.79 | 24.56±4.96 | 56.58±41.58 | 4.75±1.19^{ΔΔ} | 57.50±4.00^{ΔΔ} |
| breviscapine tablet | 40mg | 12 | 92.49±34.44 | 26.58±3.81 | 69.74±33.82 | 3.90±0.96 | 48.93±7.22^{**} |
| A1 | 40mg | 12 | 93.04±35.64 | 25.75±5.26 | 75.96±35.87 | 3.95±0.84 | 48.22±5.55^{**} |
| A1 | 20mg | 11 | 92.41±42.37 | 25.90±3.51 | 78.17±28.14 | 4.77±1.11 | 51.20±7.41^{*} |

| | | | | | | | |
|---|---|---|---|---|---|---|---|
| Compared with the sham group: ^{Δ}P < 0.05, ^{ΔΔ}P < 0.01; Compared with the modeling group: *P<0.05, **P<0.01. | | | | | | | |

### 3.2 Effect on MCAO induced focal cerebral ischemia in rats

### 3.2.1 Effect on nerve dysfunction and range of cerebral infarction caused by MCAO in rats

After modeling of MCAO, rat had reduced activities and expressed movement disorders at different degrees. The modeling group differed from the sham group significantly. Each administration group was able to significantly improve the nerve dysfunction of MCAO rats and narrow the range of cerebral infarction. For A1 group, the ranges of cerebral infarction were decreased by 64.64% and 59.10%, respectively. The results were summarized in table 4.

**Table 4 Effect of A1 on nerve dysfunction and range of cerebral infarction caused by MCAO in rats**

| group | dosage (/kg) | number of animals | range of cerebral infarction | | behavior score (X±SD) | |
|---|---|---|---|---|---|---|
| | | | (X±SD, %) | rate of decrease | 6h | 24h |
| sham | 10ml | 10 | 0.04±0.09 | - | 0.6±0.97 | 0.60±1.08 |
| modeling | 10ml | 11 | 5.77±2.59^{ΔΔ} | - | 8.73±1.01^{ΔΔ} | 7.54±1.29^{ΔΔ} |
| nimodipine | 50mg | 12 | 2.27±2.56^{**} | 60.66 | 6.50±1.68^{**} | 4.83±1.40^{**} |
| breviscapine tablet | 40mg | 11 | 2.70±1.42^{**} | 53.21 | 6.27±1.01^{**} | 5.18±1.33^{**} |
| A1 | 40mg | 12 | 2.04±1.43^{**} | 64.64 | 5.75±1.42^{**} | 4.25±1.60^{**} |
| A1 | 20mg | 12 | 2.36±1.81^{**} | 59.10 | 6.42±1.62^{**} | 4.92±1.31^{**} |

| | | | | | | |
|---|---|---|---|---|---|---|
| Compared with the sham group: ^{ΔΔ}P < 0.01; Compared with the modeling group: *P<0.05, **P < 0.01. | | | | | | |

### 3.2.2 Effect on ADP-induced platelet aggregation and coagulation in MCAO rats

After modeling of MCAO in rats, platelet aggregation rate was significantly increased, clotting time decreased somewhat, and the content of fibrinogen had a trend of increase. Both samples were able to significantly inhibit ADP-induced platelet aggregation, and had a trend of decreasing the content of fibrinogen. A1 had a trend of increasing APTT time and TT time. The results were summarized in table 5.

**Table 5 Effect of A1 on coagulation and platelet aggregation in MCAO rats (X±SD)**

| group | dosage (/kg) | number of animals | indicators of coagulation | | | | rate of platelet aggregation (%) |
|---|---|---|---|---|---|---|---|
| | | | TT (s) | PT (s) | APTT (s) | FIB (g/L) | |
| sham | 10ml | 10 | 87.96±29.56 | 28.02±5.49 | 81.63±39.80 | 3.70±0.58 | 46.30±10.97 |
| modeling | 10ml | 11 | 71.58±35.64 | 25.82±7.79 | 57.86±32.72 | 4.50±1.40 | 60.38±9.28^{ΔΔ} |
| nimodipine | 50mg | 12 | 88.55±32.24 | 23.38±8.23 | 61.02±44.79 | 3.52±1.14 | 54.15±7.76 |
| breviscapine tablet | 40mg | 11 | 98.07±36.83 | 26.78±3.51 | 74.79±26.26 | 3.24±0.90^{*} | 49.5±6.80^{**} |
| A1 | 40mg | 12 | 62.04±40.80 | 23.43±8.66 | 75.15±27.41 | 3.59±0.76 | 52.38±5.24^{*} |
| A1 | 20mg | 12 | 95.55±24.80 | 25.56±5.30 | 62.48±29.08 | 3.38±1.07 | 50.52±4.07^{**} |

| | | | | | | | |
|---|---|---|---|---|---|---|---|
| Compared with the sham group: ^{Δ}P < 0.05, ^{ΔΔ}P < 0.01; Compared with the modeling group: *P < 0.05, **P<0.01. | | | | | | | |

### 3.2.3 Effect on AST, LDH and body weight of MCAO rats

For the modeling group, activities of serum AST and LDH in rats significantly increased, while body weight at 24 hour after surgery significantly decreased. For the two administration groups, the release of LDH was significantly decreased. A1 was also able to decrease the activity of AST and act significantly against the weight loss in modeling rats. These suggested both samples could significantly relieve damage to cerebral tissue. The results were summarized in table 6.

**Table 6 Effect of A1 on serum AST, LDH and body weight of MCAO rats**

| group | dosage (/kg) | number of animals | body weight (X±SD, g) | | | | serum enzymes (X±SD, u/L)) | |
|---|---|---|---|---|---|---|---|---|
| | | | prior to administration | prior to surgery | 6h after surgery | 24h after surgery | AST | LDH |
| sham | 10ml | 10 | 258.0±15.5 | 269.5±16.6 | 258.0±15.8 | 255.5±16.6 | 156.0±51.0 | 566.1±191.2 |
| modeling | 10ml | 11 | 257.3±10.1 | 267.7±9.6 | 252.3+7.5 | 240.0±8.7^{Δ} | 246.6±121.5^{Δ} | 907.1±256.7^{ΔΔ} |
| nimodipine | 50mg | 12 | 259.6±14.5 | 265.8±12.9 | 257.1±11.4 | 249.6±10.5^{*} | 198.6±118.2 | 536.7±262.8^{**} |
| breviscapine tablet | 40mg | 11 | 259.1±13.0 | 267.7±19.4 | 256.8±15.7 | 247.3±15.4 | 244.3±183.6 | 719.5±133.4^{*} |
| A1 | 40mg | 12 | 256.3±13.3 | 265.8±12.4 | 253.8±12.1 | 244.2±12.2 | 152.9±33.0^{*} | 472.0±231.9^{**} |
| A1 | 20mg | 12 | 257.9±11.6 | 268.8±12.4 | 258.3±11.9 | 250.0±15.1^{*} | 171.2±71.6 | 571.1±287.5^{**} |

| | | | | | | | | |
|---|---|---|---|---|---|---|---|---|
| Compared with the sham group: ^{Δ}P < 0.05, ^{ΔΔ}P < 0.01; Compared with the modeling group: *P<0.05, **P<0.01. | | | | | | | | |

### 4. Conclusions

An animal model which is similar to human diseases and has a good repeatability is a basis for evaluation of drug therapeutic effect. Two animal models similar to human diseases were adopted in the preset study to determine multiple pharmacodynamics indicators, and evaluate the effect of two samples on acute cardio- and cerebral-ischemia. The results showed that, A1 was able to significantly improve the ECG change of rats with myocardial ischemia caused by coronary artery ligation, decrease the activities of serum CK-MB and LDH, narrow the range of myocardial infarct, inhibit platelet aggregation, and had a trend of increasing clotting time and decreasing fibrinogen, these suggested A1 had a significant protection on myocardial ischemia. A1 could not only decrease the range of cerebral infarct of MCAO rats to prominently improve behavior disorders and significantly decrease the activities of LDH and AST in serum; but also could significantly inhibit ADP-induced platelet aggregation, and showed a certain trend of anticoagulation and thus inhibit thrombosis. The results of the study showed that A1 had a prominent protection on myocardial ischemia.

Based on the two models of ischemic cardio-cerebro vascular diseases adopted, A1 showed a prominent protection under experimental conditions, high dosage of A1 showed a better effect than 40mg/kg breviscapine tablet and 50mg/kg nimodipine tablet.

### Example 2 Comparative study on solubilities of compound A1 of the present invention and scutellarin

**Table 7 Comparison on solubilities of compound A1 of the present invention and scutellarin**

| compound | H₂O | MeOH | 95%EtOH | n-BuOH | 0.01N NaOH |
|---|---|---|---|---|---|
| scutellarin | very slightly soluble | slightly soluble | slightly soluble | slightly soluble | soluble |
| A1 | sparing soluble | soluble | soluble | soluble | soluble |

| | | | | | |
|---|---|---|---|---|---|
| Note: the definitions of very soluble, freely soluble, soluble, slightly soluble, sparing soluble, very slightly soluble, practically insoluble and insoluble accord with the China Pharmacopeia 2005. | | | | | |

As can be seen from table 7, compared with scutellarin, compound A1 of the present invention is significantly improved with respect to biological physico-chemical properties, i.e., water solubility and fat solubility thereof are both significantly improved, and thus the availability thereof can be significantly improved and accordingly the therapy effect is improved.

Use of the compound of the present invention in the manufacture of pharmaceutical preparations will be illustrated by the following examples.

### Preparation Example 1: preparation of A1 freeze-dried powder for injection

| formula: (for 1000 dosage units, specification: 50mg/dosage unit) | |
|---|---|
| 4',5,6-trimethoxyscutellarin | 50g |
| mannitol | 100g |

The preparation method is as follows:

4',5,6-trimethoxyscutellarin prepared according to preceding Preparative Example was weighed according to the above formula, and 1000 ml water for injection was added and stirred, A1 was dissolved by adjusting with 0.01 N NaOH. After A1 was dissolved, 100g of mannitol was added and stirred to dissolve A1 completely. Water for injection was added to a total liquid volume of 2000ml, then activated charcoal for injection was added in an amount of 1% of the total liquid volume and an adsorption treatment was performed for 30 minutes. Firstly the activated charcoal was removed by filtration with filter paper and ceramic rod, then the filtrate was filtered by passing through a filter membrane of 0.45µm. The filtrate was filtered finely with a filter membrane of lower than 0.2µm under a cleanliness of 100 class. The fine filtrate was transferred to a filling machine and filled in 2.0ml per unit. A stopper was half-pressed, and the filtrate was sent to a freeze vacuum dryer. The filtrate was cooled quickly to below -40 °C and kept for 2 hours. Vacuum-pumping was performed to below about 1Pa, the temperature was lifted slowly to 30 and kept for 2 hours. A stopper was pressed, the product was taken out, capped and packaged. After examined qualified, 1000 units of final products were obtained.

### Preparation Example 2: preparation of A1 dripping pills

| formula: (for 1000 pills, specification: 40mg/pill) | |
|---|---|
| A1 | 40g |
| polyethylene glycol-6000 | 200g |

The preparation method is as follows:

200g of polyethylene glycol-6000 was weighed and placed into water bath at about 100°C to melt. After melt, the system was kept a constant temperature of 65~75°C. 40g of smashed A1 was weighed and added gradually to the constant-temperature solution of polyethylene glycol-6000. The system was stirred fully to mix evenly, and then was sent to a storage tank of a dripping pill machine. After mixing evenly by stirring, dripping may be performed at a constant temperature of 65~80 °C. Dimethyl silicone oil or liquid paraffin was used as a cooling liquid. The temperature of the cooling liquid was 3.5~11°C and dripping rate was controlled at 60-80 drops/minute. After dripping, the product was taken out and the silicone oil or liquid paraffin on the surface was removed. After natural drying and stiffen at room temperature, the product was dried at 50±2°C and A1 dripping pills for oral administration were obtained.

### Preparation Example 3: preparation of A3 tablets

| formula: (for 1000 tablets, specification: 10mg/tablet) | |
|---|---|
| A1 | 10g |
| lactose | 18g |
| low substituted hydroxypropyl cellulose (L-HPC) | 50g |
| sodium carboxymethyl starch (CMS-Na) | 5g |
| ethanol | 30ml |
| water | 54ml |
| fine silica powder | 5g |

The preparation method is as follows:

Smashed A1, lactose, low substituted hydroxypropyl cellulose, sodium carboxymethyl starch and the like were weighed in proportion, treated according to conventional process, then mixed evenly, to which a suitable amount of ethanol was added as a wetting agent to prepare the damp mass. After granulation, dryness, and granule modification, the granules were sampled to examiner to be qualified. The glidant fine silica powder was added and mixed evenly, then tablets were pressed. After examined to be qualified, the products were subpackaged, labeled, and packaged, then oral disintegrating tablets of A1 were obtained.

The above examples are merely used to further illustrate the present invention without restricting it. Various modifications and changes made on the specific embodiments and applications within the scope of the present invention will fall within the protection scope of the present invention.

## Claims

1. A scutellarin derivative of formula (I): 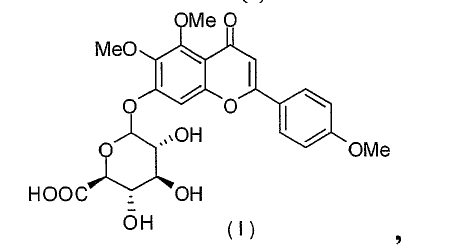 or a pharmaceutically acceptable salt thereof.

2. A process for preparing the scutellarin derivative of formula (I) according to claim 1, **characterized in that** the process comprises the following steps:
a) dissolving breviscapine in an organic solvent;
b) adding a methyl-etherification reagent and a base to the above organic solvent under stirring at 0 to 40 °C, preferably at room temperature;
c) extracting and isolating the product obtained from step b) to obtain the scutellarin derivative of formula (I).

3. The process according to claim 2, **characterized in that** the organic solvent in step a) is selected from the group consisting of N,N-dimethylformamide, dimethylsulfoxide, dichloromethane, dichloroethane and any combination thereof.

4. The process according to claim 3, **characterized in that** the organic solvent is a mixed solvent of N,N-dimethylformamide and dichloromethane in a volume ratio of 10:1 to 10:5, preferably 10:2 to 10:4, more preferably 10:3.

5. The process according to claim 3, **characterized in that** the organic solvent is a mixed solvent of dimethylsulfoxide and dichloroethane in a volume ratio of 10:1 to 10:5, preferably 10:2 to 10:4, more preferably 10:3.

6. The process according to any one of claims 2 to 5, **characterized in that** the amount of the organic solvent used in step a) is 10-100 ml per gram of breviscapine, preferably 20-80 ml per gram of breviscapine, more preferably 30-50 ml per gram of breviscapine.

7. The process according to any one of claims 2 to 6, **characterized in that** the methyl-etherification reagent in step b) is selected from the group consisting of halomethane, dimethyl sulfate and dimethyl carbonate, preferably halomethane, such as methyl iodide, methyl bromide and the like, most preferably methyl iodide, and the amount thereof in molar ratio is 1-10:1, preferably 3-5:1, more preferably 4:1, based on the amount of the reactant.

8. The process according to any one of claims 2 to 7, **characterized in that** the base in step b) is M₂CO₃ or MHCO₃, wherein M is Na, K or Li, and the amount in molar ratio of the base used is 1-15:1, preferably 3-10:1, more preferably 5-8:1, based on the amount of the reactant.

9. The process according to any one of claims 2 to 9, **characterized in that** step c) is as follows: an isolation using silica column chromatography with ethyl acetate/methanol in a volume ratio of 1-15:1, preferably 3-10:1, more preferably 6-8:1 is performed to obtain the scutellarin derivative of formula (I).

10. A pharmaceutical composition for preventing, improving or treating cardio-cerebro vascular diseases, inflammation and/or tumors, **characterized in that** the pharmaceutical composition comprises therapeutically effective amount of scutellarin derivative of formula (I) or a pharmaceutically acceptatble salt thereof according to claim 1 as an active ingredient, together with a pharmaceutically acceptatble carrier.

11. The pharmaceutical composition according to claim 10, **characterized in that** the pharmaceutical composition is formulated into conventional preparations including oral preparations, injection preparations or nasal spay according to conventional formulation method.

12. Use of the scutellarin derivative of formula (I) or a pharmaceutically acceptatble salt thereof according to claim 1 in the manufacture of a medicament for preventing, improving or treating cardio-cerebro vascular diseases, inflammation and/or tumors.
